# EUROPEAN PATENT SPECIFICATION

(11) **EP 4 228 644 B1**
(45) Date of publication and mention of the grant of the patent: **27.05.2026**
(21) Application number: 21880808.7
(22) Date of filing: 07.10.2021
(51) Int. Cl.: A61K 31/496, A61K 31/5386, A61K 31/546, A61K 31/395, A61K 31/704, A61K 31/444, A61K 31/55, A61K 45/06, A61P 31/14, A61K 31/40, A61K 31/4709, A61K 31/522, A61K 38/07, A61K 38/12

(54) **N-[4-[1-(1,4-DIOXASPIRO[4.5]DEC-8-YL)-4-(8-OXA-3-AZABICYCLO[3.2.1]OCT-3-YL)-1H-PYRAZOLO[3,4-D]PYRIMIDIN-6-YL]PHENYL]-N'-METHYLUREA FOR USE IN THE TREATMENT OF COVID19**
N-[4-[1-(1,4-DIOXASPIRO[4.5]DEC-8-YL)-4-(8-OXA-3-AZABICYCLO[3.2.1]OCT-3-YL)-1H-PYRAZOLO[3,4-D]PYRIMIDIN-6-YL]PHENYL]-N'-METHYLUREA ZUR VERWENDUNG BEI DER BEHANDLUNG VON COVID19
N-[4-[1-(1,4-DIOXASPIRO[4.5]DEC-8-YL)-4-(8-OXA-3-AZABICYCLO[3.2.1]OCT-3-YL)-1H-PYRAZOLO[3,4-D]PYRIMIDIN-6-YL]PHENYL]-N'-METHYLURÉE POUR UNE UTILISATION DANS LE TRAITEMENT DE LA COVID19

(30) Priority: 15.10.2020 US 202063092321 P
(43) Date of publication of application: 23.08.2023
(60) Divisional application: 26161433.3
(73) Proprietor: Nantcell, Inc., Culver City, California 90232 (US)
(72) Inventor: BUZKO, Oleksandr, Culver City, California 90232 (US); TANAKA, Shiho, Culver City, California 90232 (US); NIAZI, Kayvan, Culver City, California 90232 (US)
(74) Representative: J A Kemp LLP
(86) International application number: PCT/US2021/053914
(87) International publication number: WO 2022/081406

(56) References cited:
- CN-A- 111 544 442
- JIAO-FENG HUANG ET AL: "Fatal outcome in a liver transplant recipient with COVID-19", AMERICAN JOURNAL OF TRANSPLANTATION, BLACKWELL MUNKSGAARD, DK, vol. 20, no. 7, 4 May 2020 (2020-05-04), pages 1907 - 1910, XP072348468, ISSN: 1600-6135, DOI: 10.1111/AJT.15909
- ALHARTHY ABDULRAHMAN ET AL: "COVID-19 in a patient with a flare of systemic lupus erythematosus: A rare case-report", RESPIRATORY MEDICINE CME, ELSEVIER, AMSTERDAM, NL, vol. 31, 1 January 2020 (2020-01-01), XP086413056, ISSN: 2213-0071, [retrieved on 20201015], DOI: 10.1016/J.RMCR.2020.101252
- ZIBIAO ZHONG ET AL: "Clinical characteristics and immunosuppressant management of coronavirus disease 2019 in solid organ transplant recipients", AMERICAN JOURNAL OF TRANSPLANTATION, BLACKWELL MUNKSGAARD, DK, vol. 20, no. 7, 4 May 2020 (2020-05-04), pages 1916 - 1921, XP072348463, ISSN: 1600-6135, DOI: 10.1111/AJT.15928
- JOSE A MORILLAS ET AL: "Tocilizumab therapy in 5 solid and composite tissue transplant recipients with early ARDS due to SARS-CoV-2", AMERICAN JOURNAL OF TRANSPLANTATION, BLACKWELL MUNKSGAARD, DK, vol. 20, no. 11, 22 July 2020 (2020-07-22), pages 3191 - 3197, XP072342553, ISSN: 1600-6135, DOI: 10.1111/AJT.16080
- TARIQ KEWAN ET AL: "COVID-19 patient with immune thrombocytopenic purpura", INTERNATIONAL JOURNAL OF LABORATORY HEMATOLOGY, WILEY-BLACKWELL PUBLISHING LTD, GB; US, vol. 42, no. 6, 12 August 2020 (2020-08-12), pages e260 - e262, XP072389262, ISSN: 1751-5521, DOI: 10.1111/IJLH.13303
- WU CANRONG ET AL: "Analysis of therapeutic targets for SARS-CoV-2 and discovery of potential drugs by computational methods", ACTA PHARMACEUTICA SINICA B, vol. 10, no. 5, 1 May 2020 (2020-05-01), pages 766 - 788, XP093127452, ISSN: 2211-3835, DOI: 10.1016/j.apsb.2020.02.008
- ZAULI GIORGIO ET AL: "Rationale for Considering Oral Idasanutlin as a Therapeutic Option for COVID-19 Patients", FRONTIERS IN PHARMACOLOGY, vol. 11, 29 July 2020 (2020-07-29), CH, XP093223580, ISSN: 1663-9812, DOI: 10.3389/fphar.2020.01156
- B\'ALINT M\'ESZ\'AROS ET AL: "Short linear motif candidates in the cell entry system used by SARS-CoV-2 and their potential therapeutic implications", ARXIV.ORG, CORNELL UNIVERSITY LIBRARY, 201 OLIN LIBRARY CORNELL UNIVERSITY ITHACA, NY 14853, 21 April 2020 (2020-04-21), XP081650097
- DATABASE EMBASE [online] ELSEVIER SCIENCE PUBLISHERS, AMSTERDAM, NL; 12 May 2020 (2020-05-12), SONG L ET AL: "Screening novel inhibitors targeting SARS-CoV-2 S protein-ACE2 interaction based on molecular docking", XP002812562, Database accession no. EMB-002006989483
- SONG L ET AL: "Screening novel inhibitors targeting SARS-CoV-2 S protein-ACE2 interaction based on molecular docking", CHINESE TRADITIONAL AND HERBAL DRUGS 20200512 EDITORIAL OFFICE OF CHINESE TRADITIONAL AND HERBAL DRUGS CHN, vol. 51, no. 9, 12 May 2020 (2020-05-12), pages 2361 - 2367, ISSN: 0253-2670
- NGUYENLA XAMMY ET AL: "Discovery of SARS-CoV-2 antiviral synergy between remdesivir and approved drugs in human lung cells", BIORXIV, 18 September 2020 (2020-09-18), XP055810452, Retrieved from the Internet <URL:https://www.biorxiv.org/content/10.1101/2020.09.18.302398v1.full.pdf> [retrieved on 20210604], DOI: 10.1101/2020.09.18.302398
- MAFFUCCI IRENE ET AL: "In Silico Drug Repurposing for SARS-CoV-2 Main Proteinase and Spike Proteins", JOURNAL OF PROTEOME RESEARCH, vol. 19, no. 11, 7 September 2020 (2020-09-07), pages 4637 - 4648, XP055954029, ISSN: 1535-3893, Retrieved from the Internet <URL:http://pubs.acs.org/doi/pdf/10.1021/acs.jproteome.0c00383> DOI: 10.1021/acs.jproteome.0c00383
- WU CANRONG, LIU YANG, YANG YUEYING, ZHANG PENG, ZHONG WU, WANG YALI, WANG QIQI, XU YANG, LI MINGXUE, LI XINGZHOU, ZHENG MENGZHU, C: "Analysis of therapeutic targets for SARS-CoV-2 and discovery of potential drugs by computational methods", ACTA PHARMACEUTICA SINICA B, vol. 10, no. 5, 1 May 2020 (2020-05-01), pages 766 - 788, XP055856728, ISSN: 2211-3835, DOI: 10.1016/j.apsb.2020.02.008
- GARCIA GUSTAVO, SHARMA ARUN, RAMAIAH ARUNACHALAM, SEN CHANDANI, KOHN DONALD, GOMPERTS BRIGITTE, SVENDSEN CLIVE N., DAMOISEAUX ROBE: "Antiviral Drug Screen of Kinase inhibitors Identifies Cellular Signaling Pathways Critical for SARS-CoV-2 Replication", BIORXIV, 25 June 2020 (2020-06-25), pages 1 - 29, XP055874182, Retrieved from the Internet <URL:https://www.biorxiv.org/content/10.1101/2020.06.24.150326v1.full.pdf> [retrieved on 20211217], DOI: 10.1101/2020.06.24.150326
- SENATHILAKE KANISHKA, SAMARAKOON SAMEERA, TENNEKOON KAMANI: "Virtual Screening of Inhibitors Against Spike Glycoprotein of SARS-CoV-2: A Drug Repurposing Approach", PREPRINTS202003.0042.V2., 20 March 2020 (2020-03-20), pages 1 - 15, XP055808849, DOI: 10.20944/preprints202003.0042.v2
- BEOVIĆ BOJANA, DOUŠAK MAY, FERREIRA-COIMBRA JOÃO, NADRAH KRISTINA, RUBULOTTA FRANCESCA, BELLIATO MIRKO, BERGER-ESTILITA JOANA, AYO: "Antibiotic use in patients with COVID-19: a ‘snapshot’ Infectious Diseases International Research Initiative (ID-IRI) survey", JOURNAL OF ANTIMICROBIAL CHEMOTHERAPY., OXFORD UNIVERSITY PRESS, GB, vol. 75, no. 11, 1 November 2020 (2020-11-01), GB , pages 3386 - 3390, XP055922448, ISSN: 0305-7453, DOI: 10.1093/jac/dkaa326

## Description

### CROSS-REFERENCE TO RELATED APPLICATIONS

This application claims the benefit of priority under 35 U.S.C. §119(e) to U.S. Provisional Patent Application No. 63/092,321, filed October 15, 2020..

### BACKGROUND

Coronavirus disease 2019 (COVID19) is the multisystem disease caused by the novel sudden acute respiratory syndrome coronavirus 2 (SARS-CoV-2). In a matter of months, COVID19 was recognized to be a significant health threat to the elderly and people with pre-existing medical conditions, and later as a significant health threat to everyone. Currently, there is no known cure for COVID19. Physicians, nurses, and scientists worldwide have undertaken an unprecedented, accelerated effort in finding treatments to ease COVID19 patient suffering, mitigate severity of disease symptoms, and prevent mortal outcomes.

COVID19 may affect every system in the human body, though it was first recognized by its effect on the respiratory system. SARS-CoV-2 is thought to initially infect a subject via the upper respiratory system, and the subsequent viral infection can be asymptomatic, mildly symptomatic, or severely symptomatic. Mild symptoms include fever (>100.4 °F), cough, fatigue, body aches, chills, sore throat, runny nose, congestion, conjunctivitis, gastrointestinal, abdominal pain, neurological including depression, tingling of extremities, and new loss of taste and smell. Severe symptoms and complications include shortness of breath (pneumonia), high fever, severe cough, seizures, stroke, blood clots, persistent chest pain/pressure, dizziness, delusion, confusion, inability to stay awake, acute respiratory distress syndrome, acute kidney injury, susceptible to additional infections (viral, bacterial, and fungal), heart problems, circulatory problems, sepsis, organ failure. Ultimately, COVID19 can cause death. A vast majority of recovered patients still experience persistent symptoms, including inflamed lungs, cardiac problems, chronic fatigue, and neurological issues months after their initial infection ("long-haulers"), even for patients with initially mild infections.

### SUMMARY

SARS-CoV-2 is an enveloped, non-segmented positive sense RNA virus of approximately 29.9 kb in length. The main structural proteins encoded by translation of the RNA sequence are the spike protein (S), small envelope protein (E), membrane glycoprotein (M), and nucleocapsid protein (N), along with several non-structural proteins (nsp 1 - 16) critical for new viral replication, construction, host cell response repression, and eventual exocytosis. The protease M^{pro}, or nsp5, plays an essential role in the virus life cycle. Nsp5 autoproteolyzes to become an active protease, and is responsible for the processing of active nsps 4 - 16. Additionally, nsp5 can potentially cleave host cell immune proteins (NLRP12, TAB1) and gene expression regulators (HDAC2, TRMT1).

The instant application provides methods for treating COVID19. Inhibiting the activity of nsp5 is an attractive target within the arsenal of weapons against SARS-CoV-2 infection and COVID19 progression. The instant application contains methods of treating COVID19 with compounds that target M^{pro} (nsp5). Any references in the description to methods of treatment refer to the compounds, pharmaceutical compositions and medicaments of the invention for use in a method of treatment of the human body by therapy.

In an aspect, provided herein, is a method of treating a SARS-CoV-2 virus infection in a subject in need thereof, the method including administering to the subject an effective amount of a pharmaceutical composition containing N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl]phenyl]-N'-methylurea (WYE-132) or a salt thereof.

In embodiments, one or more additional compounds that treat SARS-CoV-2 virus infection, or symptom(s) thereof, are administered.

### BRIEF DESCRIPTION OF THE DRAWINGS

**FIG.** 1 is a table of compounds that were tested as inhibitors of M^{pro}, or nsp5, protease activity. The observed activity of each compound against M^{pro} (nsp5) at 10 µM is indicated. Published IC50 values are provided, if known.
**FIG.** 2 is a bar graph showing the percent M^{pro}, or nsp5, inhibitory activity per compound tested. The conditions for the FRET activity assay are based on Ma C, et al. Boceprevir, GC-376, and calpain inhibitors II, XII inhibit SARS-CoV-2 viral replication by targeting the viral main protease. Cell Research. (2020) 0:1-15.

### DETAILED DESCRIPTION

After reading this description, it will become apparent to one skilled in the art how to implement the present disclosure in various alternative embodiments and alternative applications. However, all the various embodiments of the present invention will not be described herein. It will be understood that the embodiments presented here are presented by way of an example only, and are not limiting. As such, this detailed description of various alternative embodiments should not be construed to limit the scope of the present disclosure as set forth herein.

Before the present technology is disclosed and described, it is to be understood that the aspects described below are not limited to specific compositions, methods of preparing such compositions, or uses thereof as such may, of course, vary. It is also to be understood that the terminology used herein is for the purpose of describing particular aspects only and is not intended to be limiting.

The detailed description divided into various sections only for the reader's convenience and disclosure found in any section may be combined with that in another section. Titles or subtitles may be used in the specification for the convenience of a reader, which are not intended to influence the scope of the present disclosure.

### Definitions

Unless defined otherwise, all technical and scientific terms used herein have the same meaning as commonly understood by one of ordinary skill in the art to which this disclosure belongs. In this specification and in the claims that follow, reference will be made to a number of terms that shall be defined to have the following meanings:

The terminology used herein is for the purpose of describing particular embodiments only and is not intended to be limiting. As used herein, the singular forms "a", "an" and "the" are intended to include the plural forms as well, unless the context clearly indicates otherwise.

"Optional" or "optionally" means that the subsequently described event or circumstance can or cannot occur, and that the description includes instances where the event or circumstance occurs and instances where it does not.

The term "about" when used before a numerical designation, e.g., temperature, time, amount, concentration, and such other, including a range, indicates approximations which may vary by ( + ) or ( - ) 10%, 5%, 1%, or any subrange or subvalue there between. Preferably, the term "about" when used with regard to an amount means that the amount may vary by +/- 10%.

"Comprising" or "comprises" is intended to mean that the compositions and methods include the recited elements, but not excluding others. "Consisting essentially of" when used to define compositions and methods, shall mean excluding other elements of any essential significance to the combination for the stated purpose. Thus, a composition consisting essentially of the elements as defined herein would not exclude other materials or steps that do not materially affect the basic and novel characteristic(s) of the claimed invention. "Consisting of" shall mean excluding more than trace elements of other ingredients and substantial method steps. Embodiments defined by each of these transition terms are within the scope of this disclosure.

The term "coronavirus" refers to an RNA virus of the subfamily *Orthocoronviridae,* family *Coronaviridae,* order *Nidovirales,* and realm of *Riboviria.*

The term "SARS" or "Sudden Acute Respiratory Syndrome" refers to a disease caused by a SARS-coronavirus.

The term "SARS-CoV-2" or "Sudden Acute Respiratory Syndrome Coronavirus 2" or "2019-nCoV" refers to the coronavirus causative of COVID19. SARS-CoV-2 is a lipid-enveloped, positive-sense, single-strand RNA virus of the genus *Betacoronavirus* of the *Orthocoronaviridae* subfamily of coronaviruses.

The term "COVID19" or "COVID-19" or "Coronavirus Disease 2019" refers to the disease caused by an SARS-CoV-2 infection in a subject.

The term "M^{pro}" or "Mpro" or "3CL^{pro}" or "C3-like main protease" or "main protease" or "nsp5 protease" refers to a trypsin/chymotrypsin-like, and picornavirus 3C-like protease that processes the SARS viral polyproteins. M^{pro}, or nsp5, also processes several host cell proteins, including removing ubiquitin from ubiquintinated proteins.

It is understood that the examples and embodiments described herein are for illustrative purposes only and that various modifications or changes in light thereof will be suggested to persons skilled in the art and are to be included within the scope of the appended claims.

One skilled in the art would understand that descriptions of making and using the complexes described herein is for the sole purpose of illustration, and that the present disclosure is not limited by this illustration.

The publications discussed herein are provided solely for their disclosure prior to the filing date of the present application.

### COVID Treatment Methods

In an aspect, provided herein, is a method of treating a SARS-CoV-2 virus infection in a subject in need thereof. In embodiments, the method includes administering to the subject an effective amount of a pharmaceutical composition containing N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl]phenyl]-N'-methylurea or a salt thereof.

In embodiments, N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl]phenyl]-N'-methylurea or salt thereof is administered in the amount of about 5 milligrams per kilogram body weight (mg/kg) to about 50 mg/kg. In some embodiments, the N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl]phenyl]-N'-methylurea or salt thereof is administered in the amount of about 5 mg/kg. In some embodiments, the N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl]phenyl]-N'-methylurea or salt thereof is administered in the amount of about 50 mg/kg. The amount may be any value or subrange within recited ranges, including endpoints. In some embodiments, the N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl]phenyl]-N'-methylurea or salt thereof is administered via intravenous injection. In some embodiments, the N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl[phenyl]-N'-methylurea or salt thereof is administered via intraperitoneal injection.

In embodiments, one or more additional agents may be administered to the subject. The one or more additional agents may treat SARS-CoV-2 infection, and/or a symptom of SARS-CoV-2 infection. In embodiments, the one or more agents include convalescent plasma, anti-SARS-CoV-2 antibodies or antibody-like molecules, tocilizumab, acalabrutinib, tofacitinib, ruxolitinib, baricitinib, anakinra, canakinumab, apremilast, mavrilimumab, sarilumab, VIR-7831, REGN-COV2, LY-CoV555, immune system modulators, interferons and interferon-like molecules, anticoagulants, prone positioning, antiviral therapeutics, remdesivir, lopinavir, ritonavir, oseltamivir, favipiravir, umifenovir, colchicine, ivermectin, antibacterial therapeutics, nitazoxanide, steroids, corticosteroids, dexamethasone, hydroxychloroquine, chloroquine, azithromycin, non-steroidal anti-inflammatories, calpain protease inhibitors, cathespin protease inhibitors, famotidine, nafamostat, camostat, ascorbic acid, zinc, RLF-100, tradipitant, LAU-7b, HFB30132A, AZD7442, CAP-1002, brilacidin, STI-1499, ebselen, disulfiram, tideglusib, carmofur, shikonin, PX-12, N3 or anti-SARS-CoV-2 vaccine. In embodiments, one or more of the recited agents may be expressly excluded.

Further disclosed herein is a method of reducing infectivity of a SARS-CoV2 virus is provided, wherein the method includes contacting the virus with N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl]phenyl]-N'-methylurea or a salt thereof.

Also disclosed is a method of reducing activity of SARS-CoV2 nsp5 protease wherein the method includes contacting the nsp5 protease with N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl]phenyl]-N'-methylurea or a salt thereof.

The following experimental results are provided for purposes of illustration and are not intended to limit the scope of the invention. The examples which do not include the compound N-[4-[l-(1,4-dioxaspiro[4.5]dec-8-yl)-4-(8-oxa-3-azabicyclo[3.2.1]oct-3- yl)-lH- pyrazolo[3,4-d]pyrimidin-6-yl]phenyl]-N'-methylurea or a salt thereof are not part of the invention.

### EXAMPLES

### EXAMPLE 1: In silico Screen of Compounds for Potential Interaction with Protease M^{pro}

Approximately 5000 compounds were screened by AMBER20 (D.A. Case, et al. (2020) AMBER 2020, University of California, San Francisco) for potential interaction with M^{pro}. A public database of compounds approved by the FDA or in phase 2/3 clinical trials was used (FDA-Approved Drug Library, cat. no. L1300 and Preclinical/Clinical Compound Library, cat. no. L3900, Selleckchem), as well as the published crystal structure of SARS-CoV-2 Mpro (RCSB Protein Databank Number 6LU7, Z. Jin, et al. Nature 582, 289-293 (2020)).

Compounds found to potentially interact by the AMBER20 analysis with M^{pro} were further evaluated using Autodock Vina (O. Trott and A.J. Olson, J. Comp. Chem. 31, 455-461 (2010)). Autodock Vina modeled the interaction of a compound with the protein and how the interaction may change the protein conformation to determine which compounds are most likely to bind to the M^{pro} protein. Compounds having a good score were further evaluated by visual inspection of the 3D compound-protein complex. Visual inspection evaluated numerous criteria, including formation of hydrogen bonds, conformation of the compound to the predicted binding site, and positioning of hydrophobic and hydrophilic constituents upon compound binding.

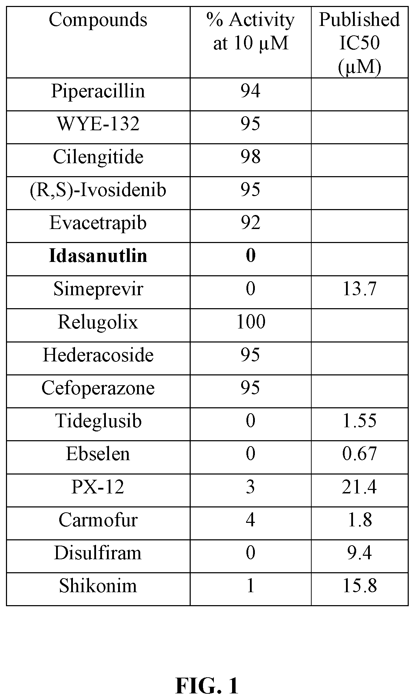

Finally, the remaining compounds were evaluated for molecular dynamics, e.g. how long the compound is expected to bind to M^{pro}. Resulting candidate compounds included piperacillin, WYE-132 (N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl]phenyl]-N'-methylurea), cilengitide, (R,S)-ivosidenib, evacetrapib, idasantulin, simeprevir, relugolix, hederacoside, and cefoperazone. See FIG. 1.

### EXAMPLE 2: FRET Assay To Determine M^{pro} Activity

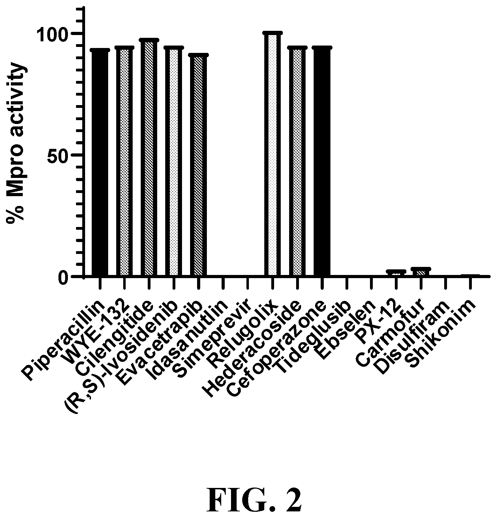

The compounds identified in Example 1 were analyzed by fluorescence resonance energy transfer (FRET) assay to determine their ability to bind and inhibit M^{pro} *in vitro.* The FRET assay conditions were adapted from Ma C, et al. Cell Research. (2020) 0:1-15. M^{pro} enzymatic activity was tested in the presence of the compounds listed in FIG. 1, and the results of the FRET activity assay are shown in FIG. 2.

Various modifications and variations of the described methods and system of the present invention will be apparent to those skilled in the art without departing from the scope of the appended claims. Although the present invention has been described in connection with specific preferred embodiments, it should be understood that the invention as claimed should not be unduly limited to such specific embodiments. Indeed, various modifications of the described modes for carrying out the invention which are obvious to those skilled in biochemistry and biotechnology or related fields are intended to be within the scope of the claims.

### REFERENCES

1. C. Ma, et al. Boceprevir, GC-376, and calpain inhibitors II, XII inhibit SARS-CoV-2 viral replication by targeting the viral main protease. Cell Res. 0, 1-15 (2020).
2. Z. Jin, et al. Structure of Mpro from SARS-CoV-2 and discovery of its inhibitors. Nature 582, 289-293 (2020).
3. R. Salomon-Ferrer, D.A. Case, R.C. Walker. An overview of the Amber biomolecular simulation package. WIREs Comput. Mol. Sci. 3, 198-210 (2013).
4. D.A. Case, T.E. Cheatham, III, T. Darden, H. Gohlke, R. Luo, K.M. Merz, Jr., A. Onufriev, C. Simmerling, B. Wang and R. Woods. The Amber biomolecular simulation programs. J. Comp. Chem. 26, 1668-1688 (2005).
5. D.A. Case, K. Belfon, I.Y. Ben-Shalom, S.R. Brozell, D.S. Cerutti, T.E. Cheatham, III, V.W.D. Cruzeiro, T.A. Darden, R.E. Duke, G. Giambasu, M.K. Gilson, H. Gohlke, A.W. Goetz, R. Harris, S. Izadi, S.A. Izmailov, K. Kasavajhala, A. Kovalenko, R. Krasny, T. Kurtzman, T.S. Lee, S. LeGrand, P. Li, C. Lin, J. Liu, T. Luchko, R. Luo, V. Man, K.M. Merz, Y. Miao, O. Mikhailovskii, G. Monard, H. Nguyen, A. Onufriev, F.Pan, S. Pantano, R. Qi, D.R. Roe, A. Roitberg, C. Sagui, S. Schott-Verdugo, J. Shen, C.L. Simmerling, N.R.Skrynnikov, J. Smith, J. Swails, R.C. Walker, J. Wang, L. Wilson, R.M. Wolf, X. Wu, Y. Xiong, Y. Xue, D.M. York and P.A. Kollman. AMBER 2020, University of California, San Francisco (2020).
6. O. Trott, A. J. Olson. AutoDock Vina: improving the speed and accuracy of docking with a new scoring function, efficient optimization and multithreading. J. Comp. Chem. 31, 455-461 (2010).
7. CN 111 544 442 A (2020).
8. Wu Canrong et al.: "Analysis of therapeutic targets for SARS-CoV-2 and discovery of potential drugs by computational methods", Acta Pharmaceutica Sinica B, 10(5):766-788, (2020)

## Claims

1. A pharmaceutical composition for use in a method of treating a SARS-CoV-2 virus infection in a subject in need thereof, the method comprising administering to the subject an effective amount of the pharmaceutical composition, said pharmaceutical composition comprising N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl]phenyl]-N'-methylurea or a salt thereof.

2. The pharmaceutical composition for use according to claim 1, wherein the N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl]phenyl]-N'-methylurea or salt thereof is administered in the amount of about 5 mg/kg to about 50 mg/kg.

3. The pharmaceutical composition for use according to claim 2,
wherein the method further comprises administering one or more agents selected from the group consisting of convalescent plasma, anti-SARS-CoV-2 antibodies or antibody-like molecules, tocilizumab, acalabrutinib, tofacitinib, ruxolitinib, baricitinib, anakinra, canakinumab, apremilast, mavrilimumab, sarilumab, VIR-7831, REGN-COV2, LY-CoV555, immune system modulators, interferons and interferon-like molecules, anticoagulants, prone positioning, antiviral therapeutics, remdesivir, lopinavir, ritonavir, oseltamivir, favipiravir, umifenovir, colchicine, ivermectin, antibacterial therapeutics, nitazoxanide, steroids, corticosteroids, dexamethasone, hydroxychloroquine, chloroquine, azithromycin, non-steroidal anti-inflammatories, calpain protease inhibitors, cathespin protease inhibitors, famotidine, nafamostat, camostat, ascorbic acid, zinc, RLF-100, tradipitant, LAU-7b, HFB30132A, AZD7442, CAP-1002, brilacidin, STI-1499, ebselen, disulfiram, tideglusib, carmofur, shikonin, PX-12, N3 and anti-SARS-CoV-2 vaccines.

## Patentansprüche

1. Pharmazeutische Zusammensetzung zur Verwendung in einem Verfahren zur Behandlung einer SARS-CoV-2-Virusinfektion bei einem Patienten, der einer solchen Behandlung bedarf, wobei das Verfahren die Verabreichung einer wirksamen Menge der pharmazeutischen Zusammensetzung an den Patienten umfasst, wobei die pharmazeutische Zusammensetzung N-[4-[1-(1,4-dioxaspiro[4.5] dec-8-yl)-4-(8-oxa-3-azabicyclo[3.2.1]oct-3-yl)-1H-pyrazolo[3,4-d]pyrimidin-6-yl]phenyl]-N'-methylharnstoff oder ein Salz davon umfasst.

2. Pharmazeutische Zusammensetzung zur Verwendung nach Anspruch 1, wobei der N-[4-[1-(1,4-10-dioxaspiro[4.5] dec-8-yl)-4-(8-oxa-3-azabicyclo[3.2.1]oct-3-yl)-1H-pyrazolo[3,4-d]pyrimidin-6-yl]phenyl]-N'-methylharnstoff oder ein Salz davon in einer Menge von etwa 5 mg/kg bis etwa 50 mg/kg verabreicht wird.

3. Pharmazeutische Zusammensetzung zur Verwendung nach Anspruch 2,
wobei das Verfahren ferner die Verabreichung eines oder mehrerer Wirkstoffe umfasst, die aus der Gruppe ausgewählt sind, bestehend aus: Rekonvaleszenzplasma, Anti-SARS-CoV-2-Antikörpern oder antikörperähnlichen Molekülen, Tocilizumab, Acalabrutinib, Tofacitinib, Ruxolitinib, Baricitinib, Anakinra, Canakinumab, Apremilast, Mavrilimumab, Sarilumab, VIR-7831, REGN-COV2, LY-CoV555, Immunsystemmodulatoren, Interferonen und Interferon-ähnlichen Molekülen, Antikoagulanzien, Bauchlage, antiviralen Therapeutika, Remdesivir, Lopinavir, Ritonavir, Oseltamivir, Favipiravir, Umifenovir, Colchicin, Ivermectin, antibakteriellen Therapeutika, Nitazoxanid, Steroiden, Kortikosteroiden, Dexamethason, Hydroxychloroquin, Chloroquin, Azithromycin, nichtsteroidalen Entzündungshemmern, Calpain-Protease-Inhibitoren, Catepsin-Protease-Inhibitoren, Famotidin, Nafamostat, Camostat, Ascorbinsäure, Zink, RLF-100, Tradipitant, LAU-7b, HFB30132A, AZD7442, CAP-1002, Brilacidin, STI-1499, Ebselen, Disulfiram, Tideglusib, Carmofur, Shikonin, PX-12, N3 und Anti-SARS-CoV-2-Impfstoffen.

## Revendications

1. Composition pharmaceutique destinée à être utilisée dans une méthode de traitement d'une infection par le virus SARS-CoV-2 chez un sujet qui en a besoin, la méthode comprenant l'administration au sujet d'une quantité efficace de la composition pharmaceutique, ladite composition pharmaceutique comprenant de la N-[4-[1-(1,4-dioxaspiro[4.5]déc-8-yl)-4-(8-oxa-3-azabicyclo[3.2.1]oct-3-yl)-1H-pyrazolo[3,4-d]pyrimidin-6-yl]phényl]-N'-méthylurée ou un sel de celle-ci.

2. Composition pharmaceutique destinée à être utilisée selon la revendication 1, dans laquelle la N-[4-[1-(1,4-dioxaspiro[4.5]déc-8-yl)-4-(8-oxa-3-azabicyclo[3.2.1]oct-3-yl)-1H-pyrazolo[3,4-d]pyrimidin-6-yl]phényl]-N'-méthylurée ou le sel de celle-ci sont administrés en une quantité d'environ 5 mg/kg à environ 50 mg/kg.

3. Composition pharmaceutique destinée à être utilisée selon la revendication 2,
ladite méthode comprenant en outre l'administration d'un ou plusieurs agents choisis dans le groupe constitué par le plasma de convalescents, les anticorps anti-SARS-CoV-2 ou les molécules de type anticorps, le tocilizumab, l'acalabrutinib, le tofacitinib, le ruxolitinib, le baricitinib, l'anakinra, le canakinumab, l'apremilast, le mavrilimumab, le sarilumab, VIR-7831, REGN-COV2, LY-CoV555, les modulateurs du système immunitaire, les interférons et molécules de type interférons, les anticoagulants, la position ventrale, les traitements antiviraux, le remdesivir, le lopinavir, le ritonavir, l'oseltamivir, le favipiravir, l'umifénovir, la colchicine, l'ivermectine, les traitements antibactériens, le nitazoxanide, les stéroïdes, les corticostéroïdes, la dexaméthasone, l'hydroxychloroquine, la chloroquine, l'azithromycine, les anti-inflammatoires non stéroïdiens, les inhibiteurs des protéases calpaïne, les inhibiteurs de protéases cathepsine, la famotidine, le nafamostat, le camostat, l'acide ascorbique, le zinc, RLF-100, le tradipitant, LAU-7b, HFB30132A, AZD7442, CAP-1002, la brilacidine, STI-1499, l'ebsélène, le disulfirame, le tideglusib, le carmofur, la shikonine, PX-12, N3 et les vaccins anti-SARS-CoV-2.
